# EUROPEAN PATENT APPLICATION

(11) **EP 4 789 680 A1**
(43) Date of publication of application: **12.08.2026**
(21) Application number: 24887298.8
(22) Date of filing: 29.04.2024
(51) Int. Cl.: A61K 38/08, C07K 5/10, C07K 7/06, A61P 19/02

(54) **NOVEL PACE4 INHIBITOR AND USE THEREOF IN ANTI-OSTEOARTHRITIS**

(30) Priority: 08.11.2023 CN 202311487117
(71) Applicant: Inflamax Pharmaceuticals Limited, Guangdong 510530 (CN)
(72) Inventor: TORTORELLA, Mickey Daniel, Guangzhou, Guangdong 510530 (CN); CHEN, Longhui, Guangzhou, Guangdong 510530 (CN); ZHANG, Huatang, Guangzhou, Guangdong 510530 (CN)
(74) Representative: Torner, Juncosa I Associats, SL
(86) International application number: PCT/CN2024/090607
(87) International publication number: WO 2025/097674

(57) **Abstract**

A novel PACE4 inhibitor and use thereof in anti-osteoarthritis. A novel PACE4 inhibitory peptide is designed and synthesized, which can effectively inhibit the expression of extracellular PACE4 without affecting normal cells, and is stable in joints and unstable in plasma, so that the PACE4 inhibitory peptide has excellent safety and can be effectively used in the prevention and treatment of OA.

## Description

### TECHNICAL FIELD

The present disclosure relates to the technical field of biomedicines, and in particular, to a novel PACE4 inhibitor and use thereof in anti-osteoarthritis.

### BACKGROUND

Osteoarthritis (OA) pathologically involves the degeneration of articular cartilage, as well as subchondral bone sclerosis and osteophyte formation, all of which lead to impaired joint function. These structural changes are often accompanied by pain, limited mobility, and joint instability. Thus, total joint replacement surgery is usually required as a treatment option. While current treatments can relieve mild to moderate pain and inflammation associated with OA, they do not protect cartilage from further damage, and such treatments have not been shown to slow disease progression. Therefore, therapeutic approaches that prevent or slow structural and functional changes in joints will effectively address a significant unmet medical need.

The main cause of cartilage loss is the protein degradation of proteoglycans and type II collagen synthesized by chondrocytes. Proteoglycans and type II collagen synthesized by chondrocytes, which are key components of the extracellular matrix of chondrocytes, account for 90% of the dry weight of the extracellular matrix. The proteoglycans synthesized by chondrocytes, which are glycosaminoglycan-containing proteoglycans, can hydrate the collagen network, thereby providing cartilage with compressibility and elasticity. Therefore, maintaining the content of proteoglycans synthesized by chondrocytes in articular cartilage is crucial for the tissue function. Loss of proteoglycans synthesized by chondrocytes is an early and critical event in the progression of cartilage destruction, which is mainly attributed to the proteolytic cleavage of aggrecan core protein by aggrecanases. Two cartilage aggrecanases, ADAMTS-4 and ADAMTS-5, have been identified as the major enzymes capable of hydrolyzing proteoglycans synthesized by chondrocytes in OA so far (see Tortorella MD, Malfait AM. Will the Real Aggrecanase(s) Step Up: Evaluating the Criteria that Define Aggrecanase Activity in Osteoarthritis. Curr Pharm Biotechnol. 2008; 9:16-23.). These metalloenzymes are synthesized as potential enzymes and require the removal of the prodomain to exert their activity. PACE4 has been identified as a proprotein convertase responsible for activating aggrecanases in OA, and therefore can serve as a novel target for the development of OA therapeutic drugs.

The known roles of PACE4 in OA include the following: PACE4 has been isolated from human OA cartilage cultures; potential recombinant proADAMTS-4 and proADAMTS-5 can be activated by recombinant PACE-4 in solution; addition of recombinant PACE-4 to human articular cartilage cultures will trigger aggrecanase-mediated catabolism similar to that observed in OA. PACE4 protein is located in the OA cartilage regions undergoing fibrosis/degradation; inhibition of PACE4 with a broad-spectrum inhibitor can block cartilage degradation in OA cartilage explants and cultures of normal human cartilage explants stimulated with inflammatory cytokines. siRNA-mediated suppression of PACE4 expression blocks cartilage degradation in inflammatory cytokine-stimulated OA and normal human cartilage cultures.

With the increasing aging of the population, the prevalence of OA worldwide is expected to continue to rise in the coming decades. Currently, the focus of OA treatment is on controlling signs and symptoms, with nonsteroidal anti-inflammatory drugs (such as COX-2 inhibitors) as the main therapeutic agents. But in fact, in the treatment of OA, the development of an effective therapy that can significantly slow or even reverse the progression of OA represents a major unmet clinical need in the current art of OA treatment. Currently, drugs considered to have cartilage-maintaining effects include diacerein, glucosamine, oxaprozin, and chondroitin sulfate. Therefore, in addition to these disease-ameliorating drugs, there remains great potential for the development of OA therapeutic drugs that can provide better pain relief effect, fewer side effects, or a higher upper limit of efficacy. Currently, no PACE4 inhibitor has been available for the intra-articular injection for the treatment of OA, which meets the requirements of selective activity against PACE4, absence of cell permeability, stability in synovial fluid, stability in the joint, instability in plasma, and water solubility.

### SUMMARY

The present disclosure aims to solve at least one of the aforementioned technical problems existing in the prior art. To this end, an object of the present disclosure is to provide a novel PACE4 inhibitor and use thereof in anti-osteoarthritis. The present disclosure designs and synthesizes a novel PACE4 inhibitory peptide that can effectively inhibit the expression of extracellular PACE4 without affecting normal cells, and is stable in joints but unstable in plasma, thus exhibiting excellent safety thereto and being effectively used in the prevention and treatment of osteoarthritis (OA).

In the present disclosure, the term "peptide" refers to an oligomer to a short polymer of amino acids linked together by peptide bonds. Unlike other amino acid polymers (e.g., proteins, polypeptides, etc.), peptides are typically about 50 amino acids or less in length (e.g., 50, 45, 40, 35, 30, 25, 20, 15, 10 or fewer, or a range therebetween (e.g., 10-30)). Peptides may comprise natural amino acids, non-natural amino acids, amino acid analogs, and/or modified amino acids. Peptides may be subsequences of naturally occurring proteins or non-natural (artificial) sequences.

The term "amino acid analog" refers to a natural or non-natural amino acid in which one or more of C-terminal carboxyl group, N-terminal amino group, and side chain functional group has been reversibly or irreversibly chemically blocked or otherwise modified into another functional group.

In a first aspect, the present disclosure provides a polypeptide or a derivative thereof, the polypeptide has a general formula as shown in formula (I):

Arg-AA2-AA3-Arg-AA4 formula (I),

wherein,
AA2 is selected from the group consisting of Phe and Ala;
AA3 is selected from the group consisting of Arg and Lys; and
AA4 is Thr, or AA4 is absent.

In some embodiments of the present disclosure, the polypeptide is a PACE4 inhibitory peptide, that is, a polypeptide having inhibitory activity on PACE4 expression.

In some embodiments of the present disclosure, the polypeptide is:

AA1-Arg-AA2-AA3-Arg-AA4;

wherein AA1 is selected from the group consisting of Arg, Pro, and a combination thereof.

In some embodiments of the present disclosure, AA1 is Arg, Pro, Arg-Pro, or Pro-Arg.

In some embodiments of the present disclosure, AA1 is Arg or Pro-Arg.

In some embodiments of the present disclosure, an amino acid in the polypeptide is in D configuration or L configuration.

In the present disclosure, by substituting partial amino acid residues in the polypeptide template (SEQ ID NO: 1) with different configurations, the inhibitory effect of the PACE4 inhibitory peptide on PACE4 can be significantly improved. Meanwhile, modification of the configuration of partial amino acids can also improve the resistance of PACE4 inhibitory peptide to peptidases, thereby imparting favorable stability to the peptide in joints.

In the present disclosure, the polypeptide has at least three binding sites for proteoglycans synthesized by chondrocytes, and an optional residue for binding the proteoglycans synthesized by chondrocytes comprises AA1, the Arg residue after AA1, AA3, and the Arg residue after AA3.

In some embodiments of the present disclosure, the residue for binding the proteoglycans synthesized by chondrocytes is at least one of the Arg residue after AA1, the Arg residue after AA3, AA1 and AA3.

In some embodiments of the present disclosure, the residues for binding the proteoglycans synthesized by chondrocytes are an Arg residue at position 2 after AA1, the Arg residue after AA3, AA1 and AA3.

In some embodiments of the present disclosure, the polypeptide or the derivative thereof comprises at least three basic amino acids.

In some embodiments of the present disclosure, at least two basic amino acids are present in AA1 and AA3. In the present disclosure, cases where the at least two basic amino acids are present in AA1 and AA3 comprise the following:
(1) AA1 is Arg, and AA3 is Arg or Lys;
(2) AA1 is Pro-Arg, and AA3 is Arg or Lys; or
(3) AA1 is Arg-Pro, and AA3 is Arg or Lys.

In some specific embodiments of the present disclosure, AA1 and AA3 are selected as follows: AA1 is Arg, and AA3 is Arg or Lys; or AA1 is Pro-Arg and AA3 is Arg or Lys.

In some embodiments of the present disclosure, a modification of an intermediate residue is present in the polypeptide or the derivative thereof.

In some embodiments of the present disclosure, the modification of an intermediate residue is a chemical modification.

In some embodiments of the present disclosure, the chemical modification includes, but is not limited to, 4-trifluoromethyl modification, 4-guanidino modification and 4-methylpiperidine modification.

In some embodiments of the present disclosure, the intermediate residue comprises any amino acid residues between the C-terminus and N-terminus of an amino acid sequence of the polypeptide.

In some embodiments of the present disclosure, the intermediate residue is AA2.

In the present disclosure, the term "pharmaceutically acceptable" means that the carrier, diluent, excipient and/or a salt thereof is chemically and/or physically compatible with other ingredients in the formulation and physiologically compatible with the recipient.

In some specific embodiments of the present disclosure, the derivative comprises a pharmaceutically acceptable salt and an isomer.

In some specific embodiments of the present disclosure, the pharmaceutically acceptable salt comprises at least one of a metal salt, an ammonium salt, a salt formed with an organic base, a salt formed with an inorganic acid, and a salt formed with an organic acid.

In some specific embodiments of the present disclosure, the salt formed with an organic acid comprises a salt formed with a basic or an acidic amino acid.

In some specific embodiments of the present disclosure, examples of the metal salt comprise: an alkali metal salt, such as a sodium salt and a potassium salt; an alkaline earth metal salt, such as a calcium salt, a magnesium salt and a barium salt; and an aluminum salt. Examples of the salt formed with an organic base comprise salts formed with the following organic bases: trimethylamine, triethylamine, pyridine, methylpyridine, 2,6-lutidine, ethanolamine, diethanolamine, triethanolamine, cyclohexylamine, dicyclohexylamine, N,N''-dibenzylethylenediamine, etc. Examples of the salt formed with an inorganic acid comprise: a salt formed with hydrochloric acid, hydrobromic acid, nitric acid, sulfuric acid, phosphoric acid, etc. Examples of the salt formed with an organic acid comprise salts formed with the following organic acids: formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, p-toluenesulfonic acid, etc. Examples of the salt formed with a basic amino acid comprise salts formed with the following basic amino acids: arginine, lysine, ornithine, etc. Examples of the salt formed with an acidic amino acid comprise salts formed with the following acidic amino acids: aspartic acid, glutamic acid, etc.

In some specific embodiments of the present disclosure, the isomer comprises an optical isomer, a stereoisomer, a regioisomer, and a geometric isomer.

In some specific embodiments of the present disclosure, the isomer is a stereoisomer or a tautomer.

In a second aspect, the present disclosure provides a peptide conjugate comprising the polypeptide or the derivative thereof described in the present disclosure and a modification moiety. The modification moiety is located at the N-terminus and/or C-terminus of the polypeptide or the derivative thereof.

In some specific embodiments of the present disclosure, the modification moiety comprises at least one of a chemical modification, a targeting moiety, a fluorescent dye and a protein tag.

In some specific embodiments of the present disclosure, the chemical modification comprises at least one of amidation, acetylation, amination, methylation, phosphorylation, glycosylation, lipidation, and ubiquitination.

In the present disclosure, the chemical modification is used to protect the polypeptide or the derivative thereof from being cleaved by peptidases.

In some specific embodiments of the present disclosure, the targeting moiety comprises at least one of a ligand, a receptor, and an antibody.

In some specific embodiments of the present disclosure, the fluorescent dye comprises FITC.

In some specific embodiments of the present disclosure, the protein tag comprises at least one of His, Flag, GST, MBP, HA, Myc, GFP, and biotin.

In some specific embodiments of the present disclosure, the peptide conjugate comprises:

Ac-R-F-K-R-NH₂

Ac-R-F-K-R-T-NH₂

Ac-R-F-K-R-dT-NH₂

Ac-R-R-F-K-R-dT-NH₂

Ac-dR-R-F-K-R-dT-NH₂

P-dR-R-F-K-R-dT-NH₂

dP-dR-R-F-K-R-dT-NH₂

Ac-R-F(4-CF₃)-K-R-T-NH₂

Ac-R-F(4-CF₃)-K-R-dT-NH₂

Ac-R-R-F(4-CF₃)-K-R-dT-NH₂

Ac-dR-R-F(4-CF₃)-K-R-dT-NH₂

P-dR-R-F(4-CF₃)-K-R-dT-NH₂

dP-dR-RF(4-CF₃)-K-R-dT-NH₂

Ac-R-A(4-Pip)-K-R-T-NH₂

Ac-R-A(4-Pip)-K-R-dT-NH₂

Ac-R-R-A(4-Pip)-K-R-dT-NH₂

Ac-dR-R-A(4-Pip)-K-R-dT-NH₂

P-dR-R-A(4-Pip)-K-R-dT-NH₂

dP-dR-R-A(4-Pip)-K-R-dT-NH₂

Ac-R-F(4-Guan)-K-R-T-NH₂

Ac-R-F(4-Guan)-K-R-dT-NH₂

Ac-R-R-F(4-Guan)-K-R-dT-NH₂

Ac-dR-R-F(4-Guan)-K-R-dT-NH₂

P-dR-R-F(4-Guan)-K-R-dT-NH₂

dP-dR-R-F(4-Guan)-K-R-dT-NH₂.

In a third aspect, the present disclosure provides a nucleic acid molecule comprising:
(1) a nucleic acid molecule encoding the polypeptide or the derivative thereof, or the peptide conjugate according to the present disclosure; or
(2) a sequence having at least 70% sequence identity with (1), and the sequence retains PACE4 inhibitory activity.

In some specific embodiments of the present disclosure, the nucleic acid molecule contains a modified base.

In some specific embodiments of the present disclosure, the modification does not affect normal function of the nucleic acid molecule, the normal function includes but not limited to: storage and transmission of genetic information and cell signal transduction.

In some specific embodiments of the present disclosure, the sequence described in (2) has at least 75%, 80%, 85%, and 90% sequence identity with (1).

In some specific embodiments of the present disclosure, the sequence described in (2) has at least 91%, 92%, 93%, 94%, 95%, 96%, 97%, 98%, and 99% sequence identity with (1).

In a fourth aspect, the present disclosure provides a biomaterial comprising or expressing any one of the polypeptides or the derivatives thereof, the peptide conjugate and the nucleic acid molecule of the present disclosure.

In some specific embodiments of the present disclosure, the biomaterial comprises an expression cassette, a vector, and a cell.

In some specific embodiments of the present disclosure, the vector includes, but is not limited to, a plasmid vector, a phage vector, a cosmid vector, YAC (yeast artificial chromosome), and BAC (bacterial artificial chromosome).

In some specific embodiments of the present disclosure, the cell includes, but is not limited to, a prokaryotic cell and a eukaryotic cell, comprising a bacteria, a fungi, an insect cell, and an animal cell.

In a fifth aspect, the present disclosure provides a method for preparing the polypeptide or the derivative thereof, or the peptide conjugate of the present disclosure, comprising obtaining the same using the biomaterial described in the present disclosure or by solid-phase synthesis.

In some specific embodiments of the present disclosure, based on the specific selection of the biomaterial, those skilled in the art can perform the treatment based on conventional practices in the art. If the biomaterial is a cell, the cell is cultured to produce the polypeptide. When the biomaterial is a vector, the vector is introduced into a cell by conventional technical means in the art. After screening for positive clones, the positive clones are used to produce the polypeptide.

In some specific embodiments of the present disclosure, exemplary methods for preparing certain PACE4 inhibitory peptides are shown, which should not be construed as limiting the scope of the present disclosure unless otherwise stated or implied from the context. Furthermore, features described in connection with various or specific embodiments should not be construed as being inapplicable to the use of other embodiments disclosed in the present disclosure, unless such exclusivity is explicitly stated or implied from the context.

In a sixth aspect, the present disclosure provides a PACE4 inhibitor comprising the peptide conjugate of the present disclosure, and a pharmaceutically acceptable excipient and/or carrier.

In the present disclosure, the term "pharmaceutically acceptable excipient and/or carrier" refers to a carrier and/or an excipient that are pharmacologically and/or physiologically compatible with the subject and the active agent, which are well known in the art (see, for example, Remington's Pharmaceutical Sciences. Edited by Gennaro AR, 19th ed. Pennsylvania: Mack Publishing Company, 1995), and includes, but is not limited to, a pH adjuster, a surfactant, an adjuvant, and an ionic strength enhancer. For example, the pH adjuster includes, but is not limited to, a phosphate buffer solution; the surfactant includes, but is not limited to, a cationic, anionic, or nonionic surfactant, such as Tween-80; the ionic strength enhancer includes, but is not limited to, sodium chloride.

In the present disclosure, various organic or inorganic carrier materials commonly used as raw materials for formulations may be used as the pharmaceutically acceptable carriers without particular limitation, and may be excipients, lubricants, binders, or disintegrants in solid formulations. Liquid formulations are formulated with solvents, solubilizers, suspending agents, isotonic agents, buffers, analgesics, etc. In addition, preservatives, antioxidants, stabilizers, colorants, sweeteners and other formulation additives may be used as needed.

In a seventh aspect, the present disclosure provides a pharmaceutical composition comprising the peptide conjugate of the present disclosure and at least one second pharmaceutically active ingredient.

In some specific embodiments of the present disclosure, the second pharmaceutically active ingredient is a drug conventionally used in the art for the treatment or adjuvant treatment of OA.

In some specific embodiments of the present disclosure, the second pharmaceutically active ingredient comprises an anti-inflammatory drug, a cartilage-nourishing agent, an analgesic, and a hormone.

In some specific embodiments of the present disclosure, the anti-inflammatory drug comprises a nonsteroidal anti-inflammatory drug, such as ibuprofen, celecoxib, loxoprofen sodium, etc.; the cartilage-nourishing agent comprises glucosamine hydrochloride, chondroitin sulfate, etc.; the analgesic comprises meloxicam, diclofenac sodium, etc.; and the hormone comprises a steroid hormone.

In an eighth aspect, the present disclosure provides use of the peptide conjugate of the present disclosure in the preparation of a drug for anti-osteoarthritis.

In some specific embodiments of the present disclosure, the drug comprises a therapeutically or prophylactically effective amount of the PACE4 inhibitory peptide or the derivative thereof of the present disclosure.

In the present disclosure, the term "treatment" generally refers to treatment and therapy for humans or animals, in which some desired therapeutic effect is achieved, for example, inhibiting disease progression, including a decrease in the rate of progression, arrest of progression, remission of the disease, improvement of the disease, and cure of the disease. The treatment also comprises treatment (i.e., prevention) as a preventative measure.

In the present disclosure, the term "prevention" in relation to a disease condition in mammals refers to preventing or delaying the onset of the disease or preventing the manifestation of clinical or subclinical symptoms thereof. In the present disclosure, the "prevention" comprises administering the drug of the present disclosure to patients who are expected to have a high risk of developing the disease due to some disease-related factors but have not yet developed the disease, or to patients who have developed the disease but have no subjective symptoms; or the drug of the present disclosure may be administered to patients who are afraid of recurrence of the disease after treatment of the disease.

Dosage of the PACE4 inhibitory peptide or the derivative thereof for use in OA according to the present disclosure is not particularly limited, as long as it is a therapeutically effective amount. In the present disclosure, the term " therapeutically effective amount" refers to an amount that brings about a therapeutic effect on the subject, for example, in a subject administered with such amount, the symptoms or condition of the disease is alleviated, reduced, or eliminated, or the progression of the symptoms or condition of the disease is delayed or inhibited, as compared with a subject not administered with such amount. The therapeutically effective amount can be determined by a doctor or professional based on factors such as age, body weight, gender, and the severity of the symptoms of the subject.

In some specific embodiments of the present disclosure, a dosage form of the drug comprises at least one of powder, tablet, granule, capsule, sustained-release formulation, solution, dry suspension, emulsion, suspension, syrup, and drop.

In some specific embodiments of the present disclosure, the dosage form of the drug comprises an injectable dosage form, such as an injection.

In some specific embodiments of the present disclosure, an applicable subject of the drug comprises a subject and a patient.

In some specific embodiments of the present disclosure, the subject comprises a non-human animal and a human.

In some specific embodiments of the present disclosure, the non-human animal comprises a research animal and a companion animal, such as a mouse, a rat, a primate, a monkey, a great ape, a chimpanzee, a canine (e.g., a dog), and a feline (e.g., a cat). The human as the patient or subject of OA disease is not limited by age or gender, and may be a child, an adult, or the elderly, wherein the child may be a newborn to 12 years old.

In some specific embodiments of the present disclosure, the anti-osteoarthritis comprises treatment and/or prevention of osteoarthritis.

In a ninth aspect, the present disclosure provides a method for treating and/or preventing osteoarthritis, comprising administering the peptide conjugates of the present disclosure to a subject in need thereof.

In some specific embodiments of the present disclosure, an administration dosage of the PACE4 inhibitory peptide or the derivative thereof is a prophylactically and/or therapeutically effective amount.

In the present disclosure, the preventive and therapeutic effects of the PACE4 inhibitory peptide or the derivative thereof of the present disclosure on osteoarthritis have been confirmed by in vitro and in vivo experiments. The paired amino acid convertase (PACE4) is expressed in degraded cartilage (extracellularly), thereby activating the proteoglycanase ADAMTS-4 and inducing proteoglycan degradation, triggering OA. The PACE4 inhibitory peptide or the derivative thereof in the present disclosure can effectively inhibit the expression of PACE4, thereby further effectively preventing and treating OA.

The beneficial effects of the present disclosure are as follows.

The PACE4 inhibitory peptide or the derivative thereof of the present disclosure exhibits excellent inhibitory effect on PACE4, with an IC₅₀ < 10 nM, and exhibits inhibitory activity with an IC₅₀ < 3 µM against GAG released by osteoblast-like cells (BNCs) stimulated by IL-1, and exerts a protective effect against cartilage degradation and reduces the expression of target biomarkers when administered to an OA surgical model.

In addition, the PACE4 inhibitory peptide or the derivative thereof of the present disclosure cannot penetrate cells, and thus mainly achieving extracellular inhibition of PACE4 without additional selective inhibition of other proprotein convertases predominantly located intracellularly, which simplifies drug design.

Moreover, the PACE4 inhibitory peptide or the derivative thereof of the present disclosure has good aqueous solubility in buffer solutions due to the presence of multiple positively charged amino acids, thereby allowing intra-articular administration at a maximum concentration of 100 µM.

Furthermore, the PACE4 inhibitory peptide or the derivative thereof of the present disclosure has favorable human pharmacokinetic/pharmacodynamic profiles, with characteristics of exhibiting stability in synovial fluid and OA cartilage (with a half-life greater than 7 days in cartilage) but instability in plasma, which enable rapid renal clearance of the parent peptide thereof and corresponding metabolites, thereby improving the safety thereof. Moreover, no irritation or toxic side effects have been found at effective doses, either by intra-articular administration or subcutaneous administration (at 100 times the effective dose).

The PACE4 inhibitory peptide or the derivative thereof of the present disclosure provides new directions and options for the development of drugs related to OA treatment, and has the potential to serve as the first intra-articularly administered OA therapeutic drug (DMOAD), achieving the treatment and prevention of OA by targeting PACE4.

### BRIEF DESCRIPTION OF DRAWINGS

FIG. 1 shows the HPLC chromatogram of Ac-R-F-K-R-NH₂.
FIG. 2 shows the mass spectrum of Ac-R-F-K-R-NH₂.
FIG. 3 shows the HPLC chromatogram of Ac-dR-R-F-K-R-dT-NH₂.
FIG. 4 shows the mass spectrum of Ac-dR-R-F-K-R-dT-NH₂.
FIG. 5 shows the HPLC chromatogram of Ac-dR-R-F(4-CF₃)-K-R-dT-NH₂.
FIG. 6 shows the mass spectrum of Ac-dR-R-F(4-CF₃)-K-R-dT-NH₂.
FIG. 7 shows the HPLC chromatogram of Ac-dR-R-A(4-Pip)-K-R-dT-NH₂.
FIG. 8 shows the mass spectrum of Ac-dR-R-A(4-Pip)-K-R-dT-NH₂.
FIG. 9 shows the HPLC chromatogram of Ac-dR-R-F(4-Guan)-K-R-dT-NH₂.
FIG. 10 shows the mass spectrum of Ac-dR-R-F(4-Guan)-K-R-dT-NH₂.
FIG. 11A shows representative stability results of the PACE4 inhibitory peptide in joint synovial fluid in the embodiments of the present disclosure.
FIG. 11B shows representative stability results of the PACE4 inhibitory peptide in plasma in the embodiments of the present disclosure.
FIG. 12 illustrates the absorption (binding) effect of the PACE4 inhibitory peptide in articular cartilage in the above embodiments.
FIGs. 13A-B show the therapeutic effects of Ac-R-F-K-R-NH₂ on the cartilage phenotype in OA mice, wherein FIG. 13A shows the therapeutic effect of the PACE4 inhibitory peptide evaluated by Safranin O-Fast Green staining of OA cartilage, and FIG. 13B shows the results of cartilage pathological scoring (OARSI, Osteoarthritis Research Society International) analysis for different groups (n=8; *p<0.05, ***p<0.001).
FIGs. 14A-B show the therapeutic effects of Ac-dR-R-F-K-R-dT-NH₂ on the cartilage phenotype in OA mice, wherein FIG. 14A shows the therapeutic effect of the PACE4 inhibitory peptide evaluated by Safranin O-Fast Green staining of OA cartilage, and FIG. 14B shows the results of cartilage pathological scoring (OARSI) analysis for different groups (n=8; *p<0.05, ***p<0.001).
FIGs. 15A-B show the therapeutic effects of Ac-dR-R-F(4-CF₃)-K-R-dT-NH₂ on the cartilage phenotype in OA mice, wherein FIG. 15A shows the therapeutic effect of the PACE4 inhibitory peptide evaluated by Safranin O-Fast Green staining of OA cartilage, and FIG. 15B shows the results of cartilage pathological scoring (OARSI) analysis for different groups (n=8; *p<0.05, ***p<0.001).
FIGs. 16A-B show the therapeutic effects of Ac-dR-R-A(4-Pip)-K-R-dT-NH₂ on the cartilage phenotype in OA mice, wherein FIG. 16A shows the therapeutic effect of the PACE4 inhibitory peptide evaluated by Safranin O-Fast Green staining of OA cartilage, and FIG. 16B shows the results of cartilage pathological scoring (OARSI) analysis for different groups (n=8); *p<0.05, ***p<0.001).
FIGs. 17A-B show the therapeutic effects of Ac-dR-R-F(4-Guan)-K-R-dT-NH₂ on the cartilage phenotype in OA mice, wherein FIG. 17A shows the therapeutic effect of the PACE4 inhibitory peptide evaluated by Safranin O-Fast Green staining of OA cartilage, and FIG. 17B shows the results of cartilage pathological scoring (OARSI) analysis for different groups (n=8; *p<0.05, ***p<0.001).
FIGs. 18A-B show the preventive effects of Ac-R-F-K-R-NH₂ on the cartilage phenotype in OA mice, wherein FIG. 18A shows the preventive effect of the PACE4 inhibitory peptide evaluated by Safranin O-Fast Green staining of OA cartilage, and FIG. 18B shows the results of cartilage pathological scoring (OARSI) analysis for different groups (n=8; *p<0.05, ***p<0.001).
FIGs. 19A-B show the preventive effects of Ac-dR-R-F(4-Guan)-K-R-dT-NH₂ inhibitory peptide on the cartilage phenotype in OA mice, wherein FIG. 19A shows the preventive effect of the PACE4 inhibitory peptide evaluated by Safranin O-Fast Green staining of OA cartilage, and FIG. 19B shows the results of cartilage pathological scoring (OARSI) analysis for different groups (n=8; *p<0.05, ***p<0.001).
FIGs. 20A-B show the effects of Ac-dR-R-A(4-Pip)-K-R-dT-NH₂ on the subchondral bone phenotype in OA mice, wherein FIG. 20A shows the results of Safranin O-Fast Green staining, and FIG. 20B shows the results of cartilage pathological scoring (OARSI) analysis for different groups (n=8; *p<0.05, ***p<0.001).
FIG. 21A shows the TRAP staining results (day 7) of the PACE4 inhibitory peptide in the arthritis prevention test in the embodiment of the present disclosure.
FIG. 21B shows the TRAP staining results (day 14) of the PACE4 inhibitory peptide in the arthritis prevention test in the embodiment of the present disclosure.

### DETAILED DESCRIPTION

The present disclosure will be further described in detail below through specific embodiments. Unless otherwise specified, the raw materials, reagents or apparatus used in the examples and comparative examples are available from conventional commercial sources or can be obtained by existing technical methods. Unless otherwise specified, the experimental or testing methods are conventional methods in the art.

In the present disclosure, a lowercase letter "d" or "D-" in the peptide sequence represents a D-configuration amino acid. Parentheses in the peptide sequence indicate modification groups. For example, F(4-CF₃) represents 4-trifluoromethyl modified phenylalanine (Phe, F), namely (S)-2-amino-3-(4-(trifluoromethyl)phenyl)propionic acid; F(4-Guan) represents 4-guanidino modified phenylalanine, namely (S)-2-amino-3-(4-guanidinophenyl)propionic acid. F(4-Pip) represents 4-methylpiperidine-modified phenylalanine, namely (S)-2-amino-3-((4-methylpiperidine)phenyl)propionic acid.

In the following examples, all polypeptides were synthesized using solid-phase peptide synthesis. Of course, based on the structural formula of each polypeptide shown in the following examples, those skilled in the art may also synthesize the polypeptides using other methods in the art, including but not limited to the solid-phase synthesis technique used in the following examples.

### Example 1: Design and preparation of PACE4 inhibitory peptide

In this example, a design principle and preparation method of a PACE4 inhibitory peptide were exemplarily provided.

Ac-RFKR-NH₂ (SEQ ID NO: 1) was synthesized by solid-phase peptide synthesis and used as a template to obtain the following PACE4 inhibitory peptides (as shown in Table 1).

**Table 1: PACE4 inhibitory peptides**

| SEQ ID NO | Modification at AA2 | Specific PACE4 Inhibitory Peptide |
|---|---|---|
| SEQ ID NO: 1 | control molecule | Ac-R-F-K-R-NH₂ |
| SEQ ID NO: 2 | Unmodified | Ac-R-F-K-R-T-NH₂ |
| SEQ ID NO: 3 | | Ac-R-F-K-R-dT-NH₂ |
| SEQ ID NO: 4 | | Ac-R-R-F-K-R-dT-NH₂ |
| SEQ ID NO: 5 | | Ac-dR-R-F-K-R-dT-NH₂ |
| SEQ ID NO: 6 | | P-dR-R-F-K-R-dT-NH₂ |
| SEQ ID NO: 7 | | dP-dR-R-F-K-R-dT-NH₂ |
| SEQ ID NO: 8 | 4-CF₃ modification | Ac-R-F(4-CF₃)-K-R-T-NH₂ |
| SEQ ID NO: 9 | | Ac-R-F(4-CF₃)-K-R-dT-NH₂ |
| SEQ ID NO: 10 | | Ac-R-R-F(4-CF₃)-K-R-dT-NH₂ |
| SEQ ID NO: 11 | | Ac-dR-R-F(4-CF₃)-K-R-dT-NH₂ |
| SEQ ID NO: 12 | | P-dR-R-F(4-CF₃)-K-R-dT-NH₂ |
| SEQ ID NO: 13 | | dP-dR-R-F(4-CF₃)-K-R-dT-NH₂ |
| SEQ ID NO: 14 | 4-Pip modification | Ac-R-A(4-Pip)-K-R-T-NH₂ |
| SEQ ID NO: 15 | | Ac-R-A(4-Pip)-K-R-dT-NH₂ |
| SEQ ID NO: 16 | | Ac-R-R-A(4-Pip)-K-R-dT-NH₂ |
| SEQ ID NO: 17 | | Ac-dR-R-A(4-Pip)-K-R-dT-NH₂ |
| SEQ ID NO: 18 | | P-dR-R-A(4-Pip)-K-R-dT-NH₂ |
| SEQ ID NO: 19 | | dP-dR-R-A(4-Pip)-K-R-dT-NH₂ |
| SEQ ID NO: 20 | 4-Guan modification | Ac-R-F(4-Guan)-K-R-T-NH₂ |
| SEQ ID NO: 21 | | Ac-R-F(4-Guan)-K-R-dT-NH₂ |
| SEQ ID NO: 22 | | Ac-R-R-F(4-Guan)-K-R-dT-NH₂ |
| SEQ ID NO: 23 | | Ac-dR-R-F(4-Guan)-K-R-dT-NH₂ |
| SEQ ID NO: 24 | | P-dR-R-F(4-Guan)-K-R-dT-NH₂ |
| SEQ ID NO: 25 | | dP-dR-R-F(4-Guan)-K-R-dT-NH₂ |

The structural formulas of the synthesized PACE4 inhibitory peptides were shown below:
SEQ ID NOs: 2-7: 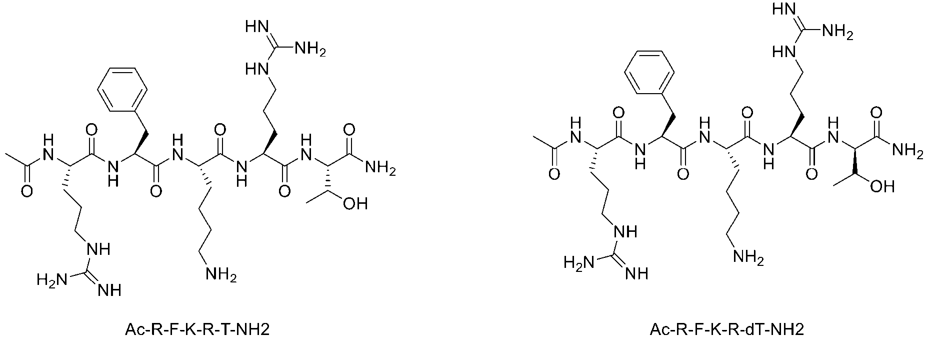 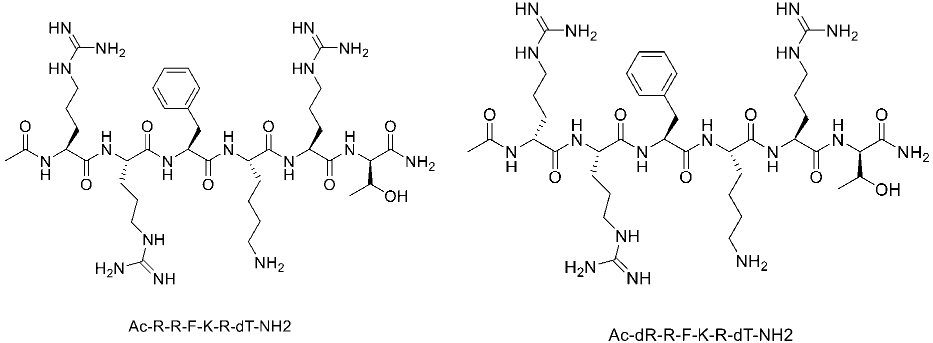 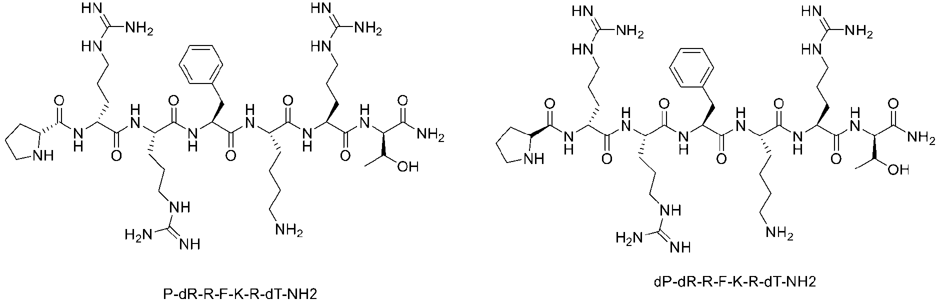
SEQ ID NOs: 8-13: 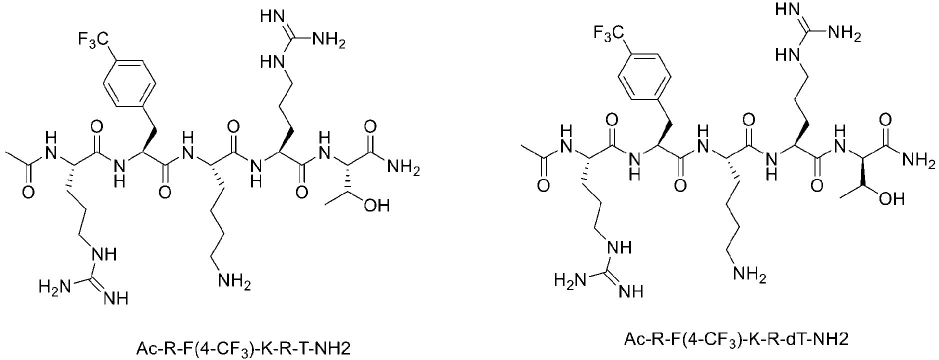 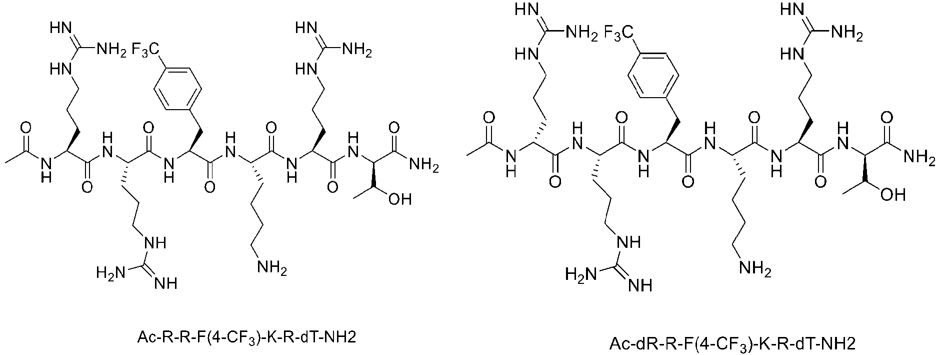 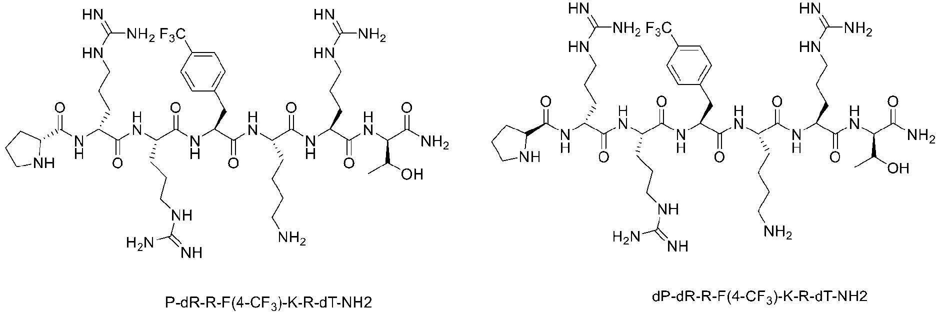
SEQ ID NOs: 14-19: 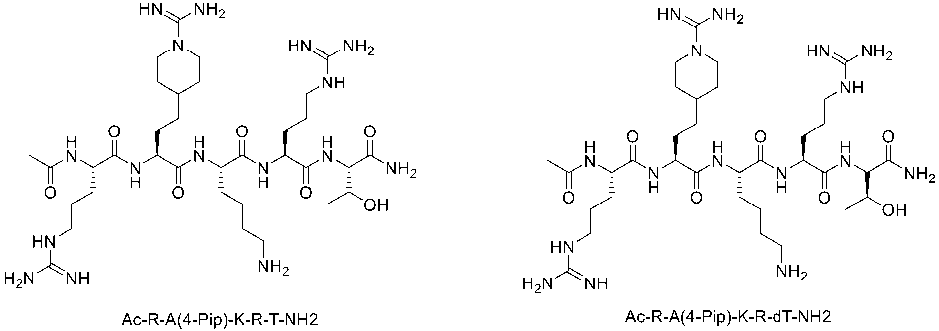 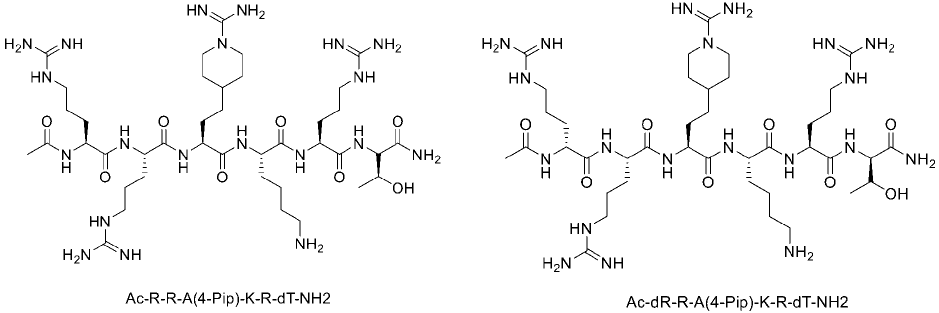 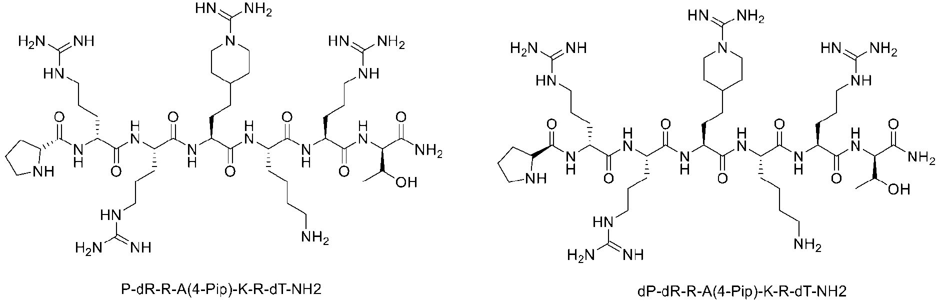
SEQ ID NOs: 20-25:

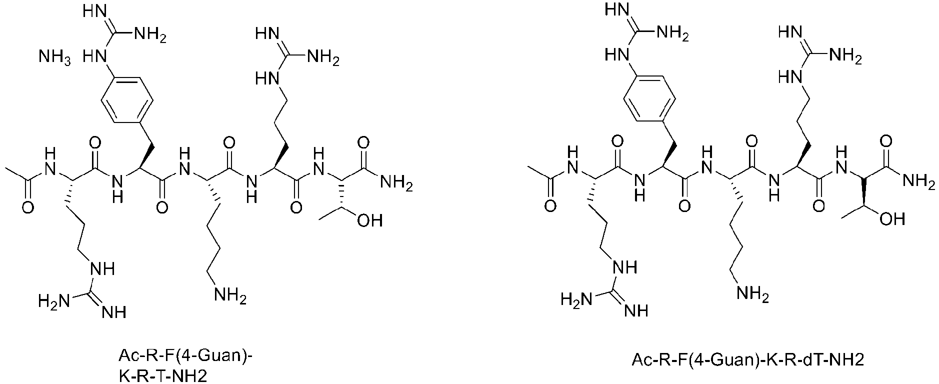

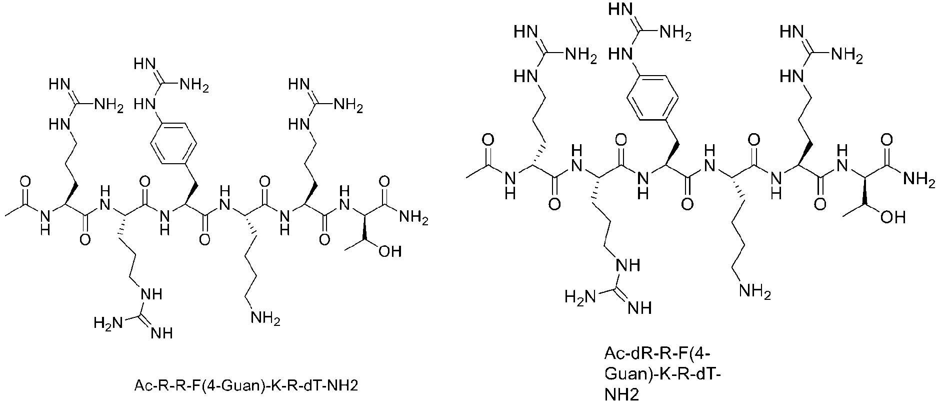

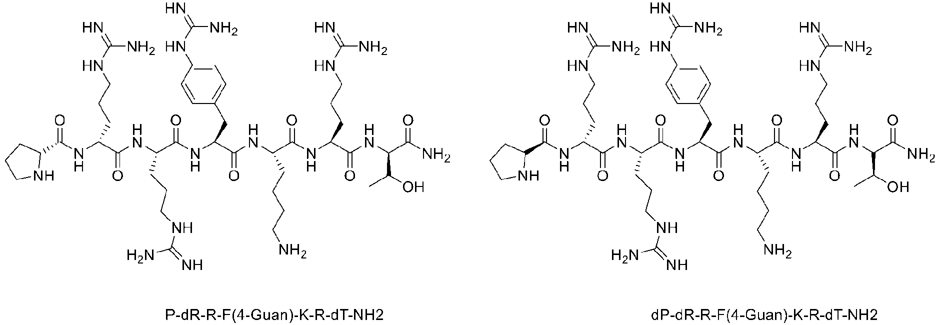

### Example 2: In vitro inhibitory effect of PACE4 inhibitory peptide

The PACE4 inhibitory peptides obtained from the above example were subjected to in vitro assay of PACE4 inhibitory effect. The specific steps were as follows: the PACE4 enzyme inhibition experiment was performed in a solution containing 20 mM Bis-Tis at pH 6.5, 1 mM CaCl₂, and 1.8 mg/mL BSA. All assays were performed using pyroGlu-Arg-Val-Lys-Arg-methyl-coumaryl-7-amide (Bachem, CA) (100 µM) as the substrate. The assay was conducted at 37°C for 60 min. A Gemini EM 96-well fluorescence spectrometer (Molecular Devices, CA) (Ex: 370 nm, Em: 460 nm) was used. The PACE4 inhibitory peptide involved in the present patent was added to the test solution at different concentrations for competitive inhibition assay to determine the IC₅₀ value of the peptide.

The results were shown in Table 2.

**Table 2: IC₅₀ of PACE4 Inhibitory Peptide against PACE4**

| Modification at AA2 | PACE4 inhibitory peptide | IC₅₀ (µM) |
|---|---|---|
| Template | Ac-R-F-K-R-NH₂ | 12.7 |
| Unmodified | Ac-R-F-K-R-T-NH₂ | 2.0 |
| | Ac-R-F-K-R-dT-NH₂ | 5.0 |
| | Ac-R-R-F-K-R-dT-NH₂ | 0.46 |
| | Ac-dR-R-F-K-R-dT-NH₂ | 0.19 |
| | P-dR-R-F-K-R-dT-NH₂ | 0.02 |
| | dP-dR-R-F-K-R-dT-NH₂ | 0.07 |
| 4-CF₃ modification | Ac-R-F(4-CF₃)-K-R-T-NH₂ | 0.74 |
| | Ac-R-F(4-CF₃)-K-R-dT-NH₂ | 0.35 |
| | Ac-R-R-F(4-CF₃)-K-R-dT-NH₂ | 0.09 |
| | Ac-dR-R-F(4-CF₃)-K-R-dT-NH₂ | 0.04 |
| | P-dR-R-F(4-CF₃)-K-R-dT-NH₂ | 0.02 |
| | dP-dR-RF(4-CF₃)-K-R-dT-NH₂ | 0.02 |
| 4-Pip Modification | Ac-R-A(4-Pip)-K-R-T-NH₂ | 0.3 |
| | Ac-R-A(4-Pip)-K-R-dT-NH₂ | 0.1 |
| | Ac-R-R-A(4-Pip)-K-R-dT-NH₂ | 0.09 |
| | Ac-dR-R-A(4-Pip)-K-R-dT-NH₂ | 0.12 |
| | P-dR-R-A(4-Pip)-K-R-dT-NH₂ | 0.07 |
| | dP-dR-R-A(4-Pip)-K-R-dT-NH₂ | 0.08 |
| 4-Guan modification | Ac-R-F(4-Guan)-K-R-T-NH₂ | 0.023 |
| | Ac-R-F(4-Guan)-K-R-dT-NH₂ | 0.021 |
| | Ac-R-R-F(4-Guan)-K-R-dT-NH₂ | 0.018 |
| | Ac-dR-R-F(4-Guan)-K-R-dT-NH₂ | 0.015 |
| | P-dR-R-F(4-Guan)-K-R-dT-NH₂ | 0.025 |
| | dP-dR-R-F(4-Guan)-K-R-dT-NH₂ | 0.034 |

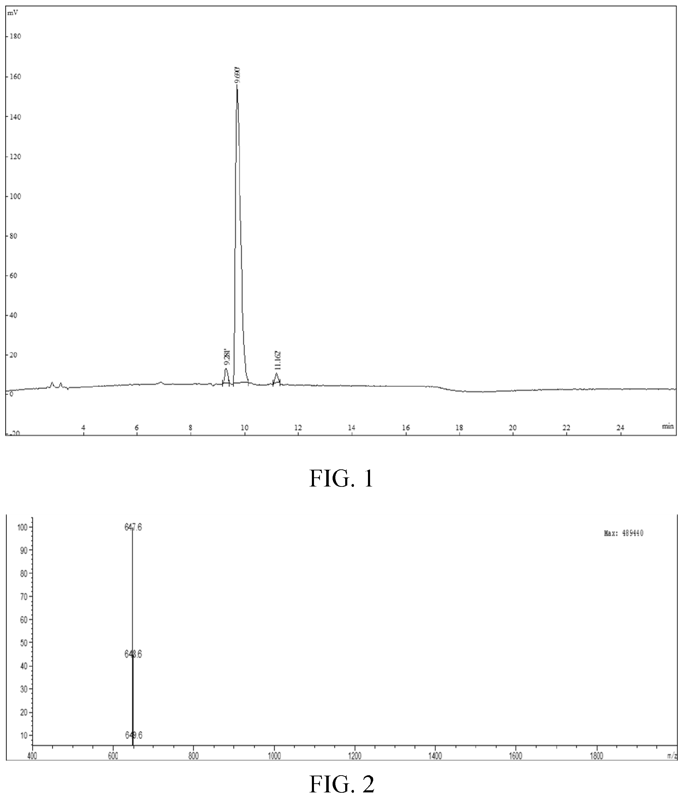

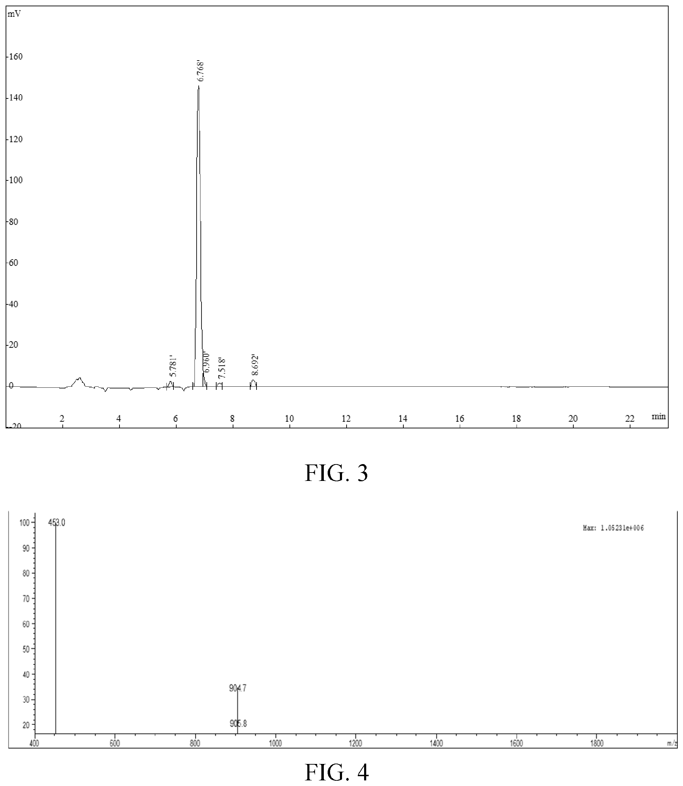

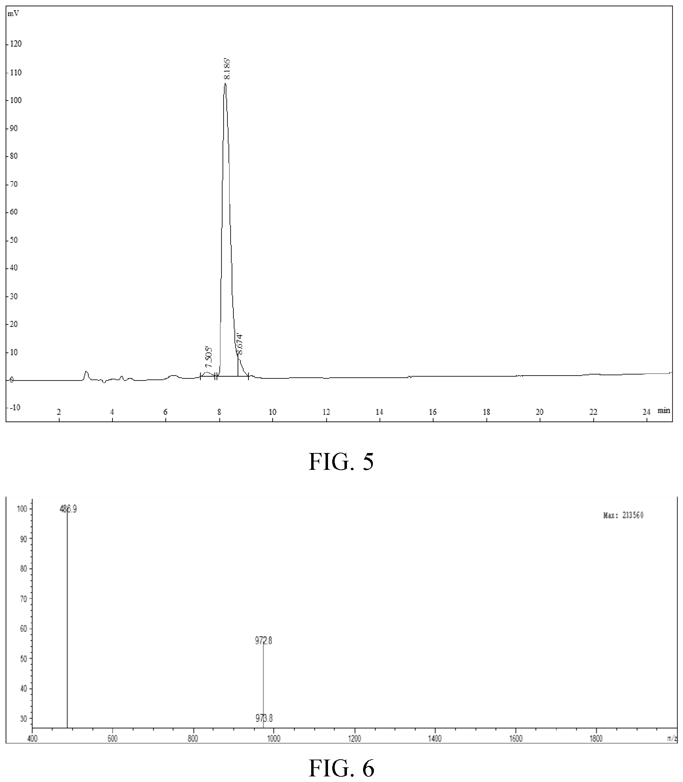

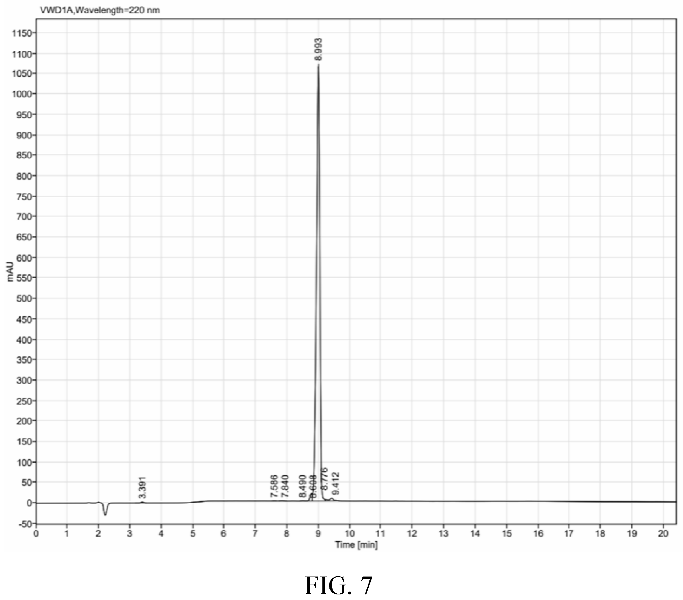

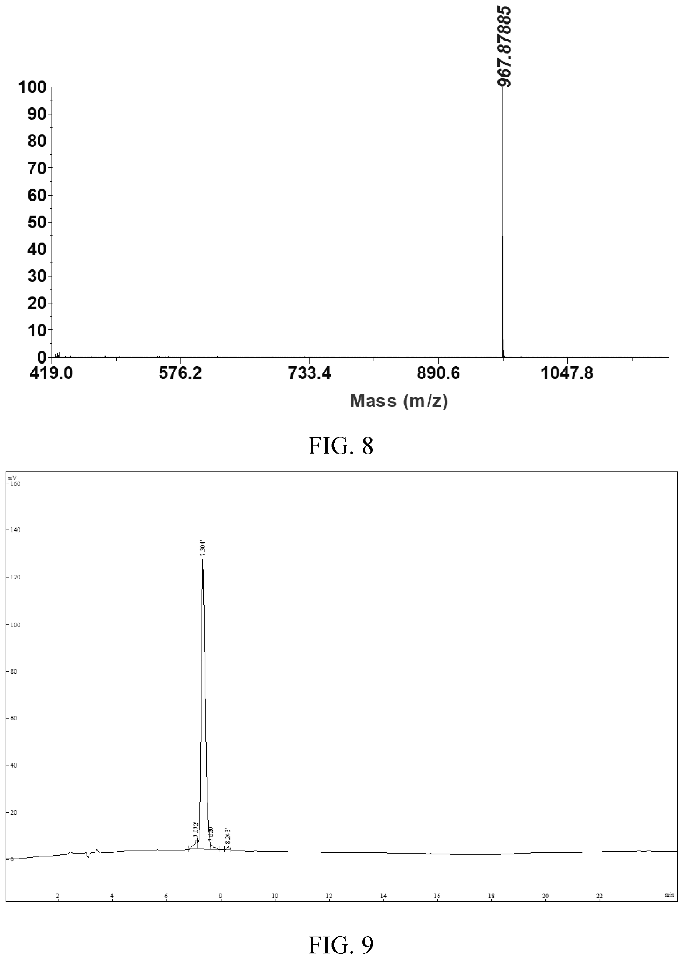

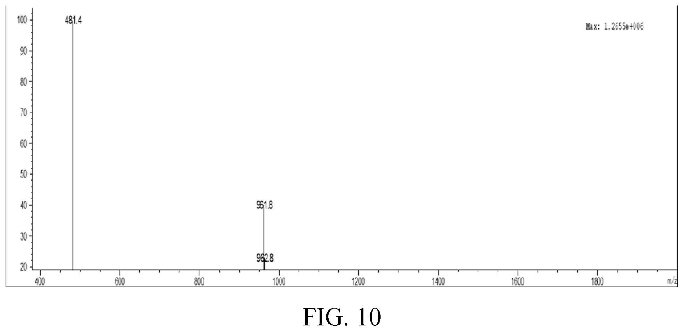

The HPLC chromatograms corresponding to each sequence were shown in FIGs. 1-10.

As could be seen from Table 1, the PACE4 inhibitory peptides obtained from the examples of the present disclosure had a significant PACE4 inhibitory effect compared with the template polypeptide. Overall, the chemical modifications at AA2 led to enhancement of the PACE4 inhibitory effect to a certain extent. In particular, the modifications at AA2 with 4-Guan, 4-CF₃ and 4-Pip resulted in significantly superior inhibitory effect relative to the unmodified version.

### Example 4: Stability of PACE4 inhibitory peptide

In this example, the PACE4 inhibitory peptides obtained above were co-incubated with human joint synovial fluid and plasma, respectively, and then HPLC was used to determine the stability after 0, 0.5 and 1 h, respectively, so as to determine the stability of the PACE4 inhibitory peptides in human joint synovial fluid and plasma.

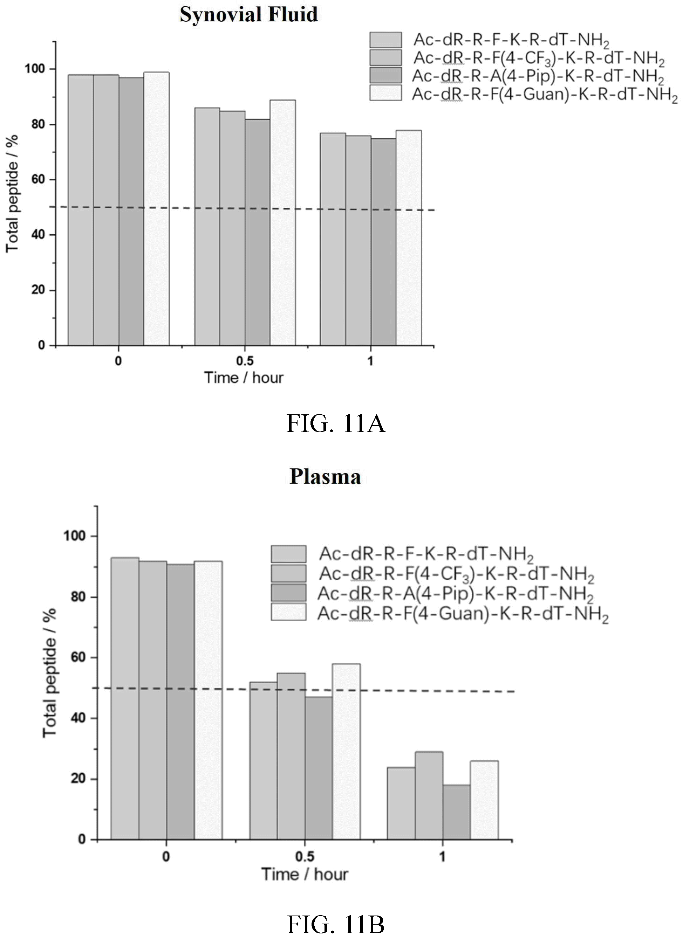

Exemplary results were shown in FIGs. 11A and 11B. It could be observed that the PACE4 inhibitory peptides in the examples of the present disclosure exhibited strong stability in human synovial fluid, but were unstable in human plasma, which was in line with the design requirements for the PACE4 inhibitory peptide in the present disclosure. The stability of the PACE4 inhibitory peptide in human synovial fluid and rapid degradation due to the instability in human plasma could effectively improve drug safety, allowing it to act only in the joints and be ineffective systemically. This effect was mainly attributed to acetylation, which protected the N-terminus of the PACE4 inhibitory peptides of the present disclosure from peptidases present in synovial fluid. Meanwhile, the C-terminal amidation group protected the C-terminus of the PACE4 inhibitory peptides from peptidase activity in synovial fluid, thereby specifically preventing internal cleavage by various peptidases in joint synovial fluid. This allowed sufficient time for the PACE4 inhibitory peptides to integrate into articular cartilage and exert their pharmacological activity. As for the PACE4 inhibitory peptides in other systems such as plasma, they were rapidly degraded, as their structural design did not confer resistance to cleavage activity under conditions of other systems.

### Example 5: Binding of PACE4 inhibitory peptide in osteoarticular joints

Another design requirement for the PACE4 inhibitory peptide in the examples of the present disclosure was as follows: on the basis of ensuring physicochemical properties of the PACE4 inhibitory peptide, the retention time and binding effect of the PACE4 inhibitory peptide in articular cartilage were improved, thereby achieving a longer-lasting therapeutic effect with fewer administrations as possible (e.g., only 4-6 administrations for treatment per year). As approximately 10-15% of cartilage consists of cartilage proteoglycans, which are rich in glycosaminoglycans, articular cartilage is negatively charged.

Based on the above theory, the PACE4 inhibitory peptides in the above examples were designed to contain at least 3 basic amino acids, such that they could directly bind to cartilage and had the inherent articular cartilage retention property. Through in vivo and in vitro assays testing the PACE4 inhibitory peptides in the above examples in cartilage cultures in vitro and administered by injection into rat knee joints, it was found that the PACE4 inhibitory peptides in the above examples could be effectively retained in cartilage for more than 2-3 weeks, and this long-term retention effect resulted in sustained therapeutic efficacy.

In addition, a binding assay was conducted on the PACE4 inhibitory peptides and articular cartilage.

15 mg of bovine nasal cartilage was collected and cultured in a culture medium at 37°C for 24 h according to conventional procedures in the art. Then, the PACE4 inhibitory peptide from the above examples was conjugated with a fluorescent moiety (5-fluorescein), added to 200 µL of the culture medium containing the above bovine nasal cartilage at a final concentration of 1,000 µM, and continuously cultured with fresh medium every day. On days 0, 1, 2, 3, 4, 5, 6 and 7 of the culture, equal aliquots of culture were collected separately, and added with 1 mL of extraction solution (containing guanidine hydrochloride at a final concentration of 4 M, 50 mM sodium acetate, pH 6.8) to extract the absorbed PACE4 inhibitory peptide from the cartilage overnight at 4°C. Finally, the fluorescence signal was detected at an excitation wavelength of 490 nm and an emission wavelength of 520 nm. The peptide concentration was quantitatively calculated based on a standard curve to determine the binding ability of the peptide with articular cartilage.

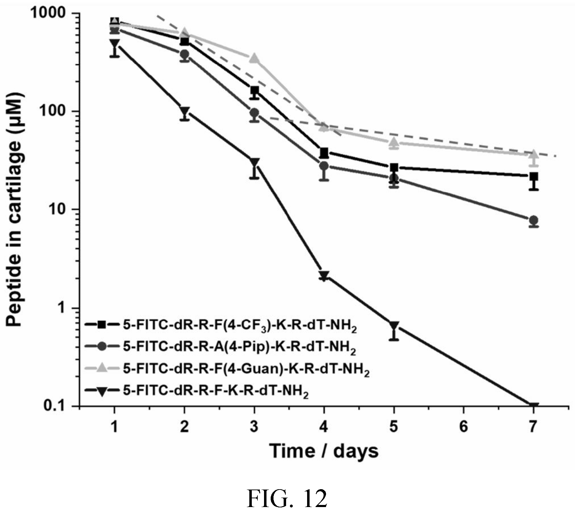

Exemplary results were shown in FIG. 12.

FIG. 12 exemplarily illustrated the absorption of two peptides representing the PACE4 inhibitory peptides in the above examples (Fluorescein-dR-R-A(4-CF₃)-K-R-dT-NH₂ and Fluorescein-dR-R-F(4-Guan)-K-R-dT-NH₂) and one control peptide (Fluorescein-R-F-G-G-dT) by articular cartilage. It could be observed that, unlike the uniform elimination of the control peptide, the PACE4 inhibitory peptides in the above examples exhibited two distinct elimination phases. The second phase of the PACE4 inhibitory peptides in the above examples exhibited a significantly slowed elimination from cartilage, indicating a very long drug half-life (t_{1/2}) in cartilage. Based on the overall assay results of all the PACE4 inhibitory peptides, it was found that the PACE4 inhibitory peptides in the above examples remained in cartilage for >7 days, with a maximum binding capacity of approximately 800 µM, and were able to rapidly bind to articular cartilage.

### Example 6: Safety and therapeutic effects of PACE4 inhibitory peptide

The PACE4 inhibitory peptide in the present disclosure was required to exhibit no local irritation or local toxicity following intra-articular injection at an effective dose in rat joints (20 µg/joint), and to cause no systemic side effects in heart, liver, kidney or other organs when administered subcutaneously at 20 mg/kg.

Meanwhile, since PACE4 is extracellularly expressed in OA cartilage but strictly restricted to the intracellular compartment in normal cartilage, the safety of the PACE4 inhibitory peptide in normal cartilage can be effectively controlled and damage to normal cartilage can be avoided by designing the PACE4 inhibitory peptide to have limited cell permeability.

In this example, a traumatic osteoarthritis model was established by performing a medial meniscus tear (MMT) surgery to verify the therapeutic effect of the PACE4 inhibitory peptides on OA. In the test for therapeutic efficacy, the injections were administered on day 3 and day 10 after the surgical modeling (i.e., a total of 2 administrations).

Specifically, in the test for therapeutic efficacy, 24 Sprague Dawley (SD) rats were divided into 3 groups (including a control group, an OA modeling group (surgery group), and an OA modeling + therapeutic administration group (surgery group + drug intervention)), with 8 rats per group. For the OA modeling + therapeutic administration group, intra-articular injections were administered on day 3 and day 10 after MMT surgery. Rats in each group were sacrificed on day 14 after modeling, and joint tissues were collected. Each PACE4 inhibitory peptide was tested individually to determine the therapeutic effect of each PACE4 inhibitory peptide. Among them, the polypeptide Ac-R-F-K-R-NH₂ (control molecule) was selected as a control example.

Cartilage tissue was collected from each group as samples. The collected cartilage tissue samples were decalcified according to conventional procedures in the art, then embedded in paraffin, and sectioned at a thickness of 7 µm. Safranin O-fast green staining was performed, and the cartilage degeneration was assessed using OARSI scoring. In addition, TRAP staining was performed to determine the subchondral bone resorption in rats before and after MMT surgery and during treatment with the PACE4 inhibitory peptide.

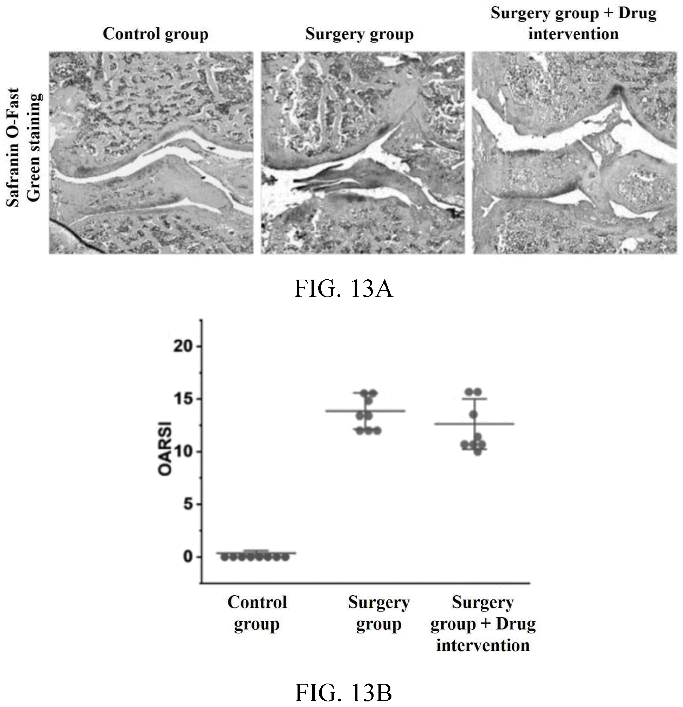

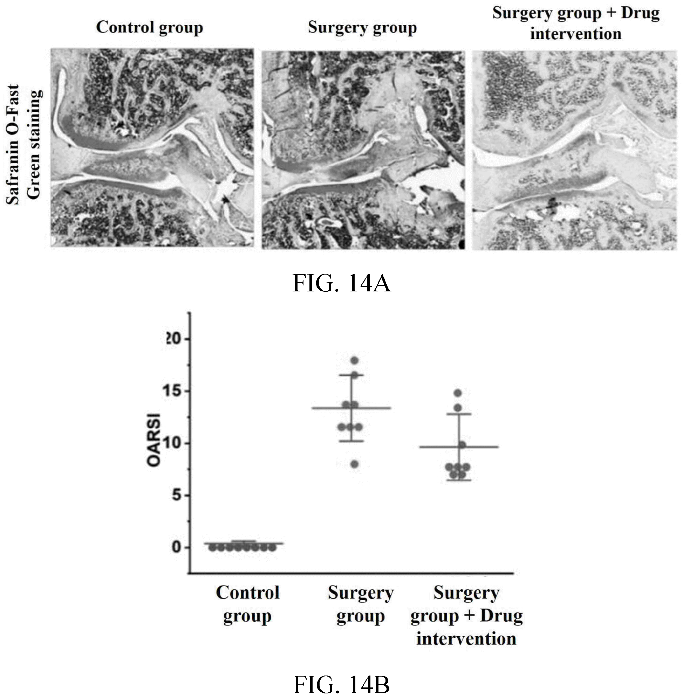

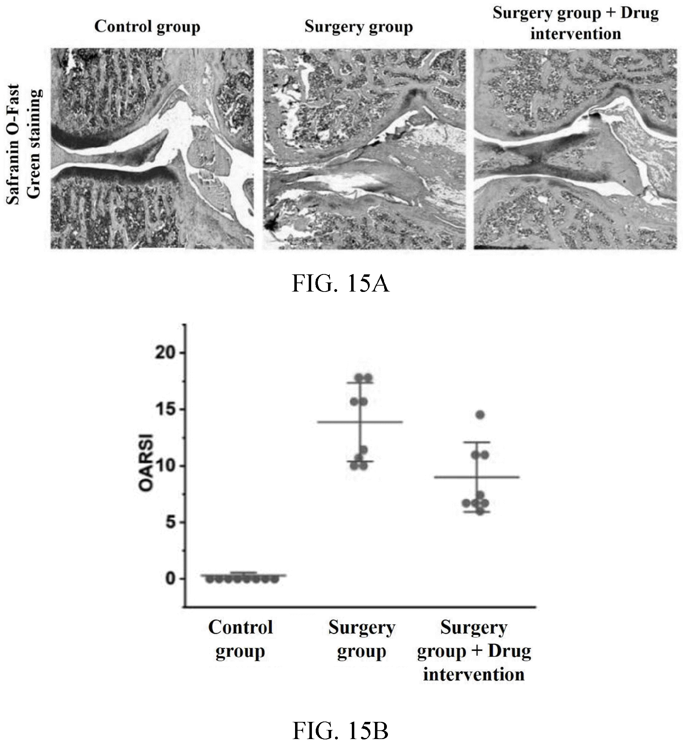

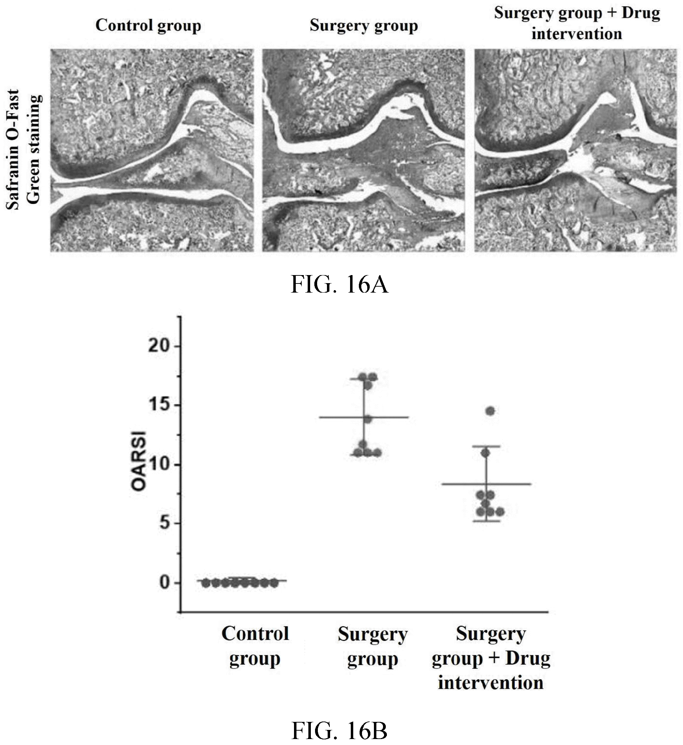

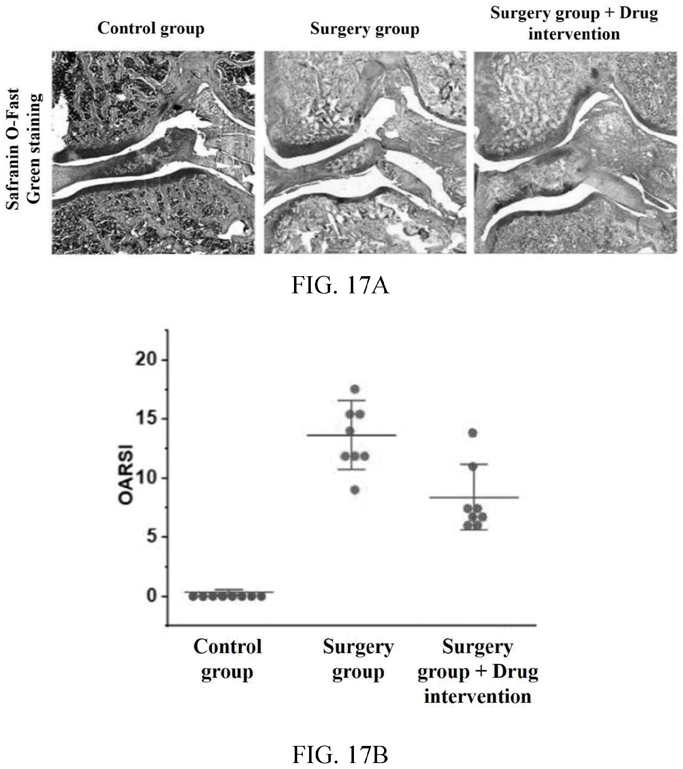

Representative results of the control molecule and several PACE4 inhibitory peptides in the examples of the present disclosure were randomly and exemplarily shown in FIGs. 13A to 17B.

It could be observed that in the test for therapeutic efficacy, Safranin O-Fast Green staining results showed that the PACE4 inhibitory peptides in the examples of the present disclosure could effectively improve surgery-induced cartilage degeneration and severe GAG (i.e., containing negatively charged carboxyl and sulfate groups, mainly released by osteoblast-like cells (BNCs) stimulated by interleukin-1 (IL-1)) loss.

### Example 7: Preventive effects of PACE4 inhibitory peptide

In this example, a traumatic osteoarthritis model was established by performing a medial meniscus tear (MMT) surgery to verify the preventive effect of the PACE4 inhibitory peptides on OA. In the test for preventive efficacy, the PACE4 inhibitory peptide was administered via intra-articular injection 3 days and 1 day before the modeling surgery (i.e., a total of 2 administrations). In the following examples, the dosage of PACE4 inhibitory peptide was 0.1 mg/kg animal body weight.

Specifically, in the test for preventive efficacy, 24 SD rats were divided into 3 groups (including a control group, an OA modeling group (surgery group), and an OA modeling + preventive administration group (surgery group + drug intervention)), with 8 rats per group. For the OA modeling + preventive administration group, intra-articular injections were administered 3 days and 1 day before MMT surgery. Rats in each group were sacrificed on day 7 after modeling, and joint tissues were collected. Each PACE4 inhibitory peptide was tested individually to determine the preventive effect of each PACE4 inhibitory peptide. Among them, the polypeptide Ac-R-F-K-R-NH₂ (control molecule) was selected as a control example.

Safranin O-Fast Green staining, OARSI scoring, and TRAP staining were performed according to the methods described in the above examples to determine the subchondral bone resorption in rats before and after MMT surgery and during treatment with the PACE4 inhibitory peptide.

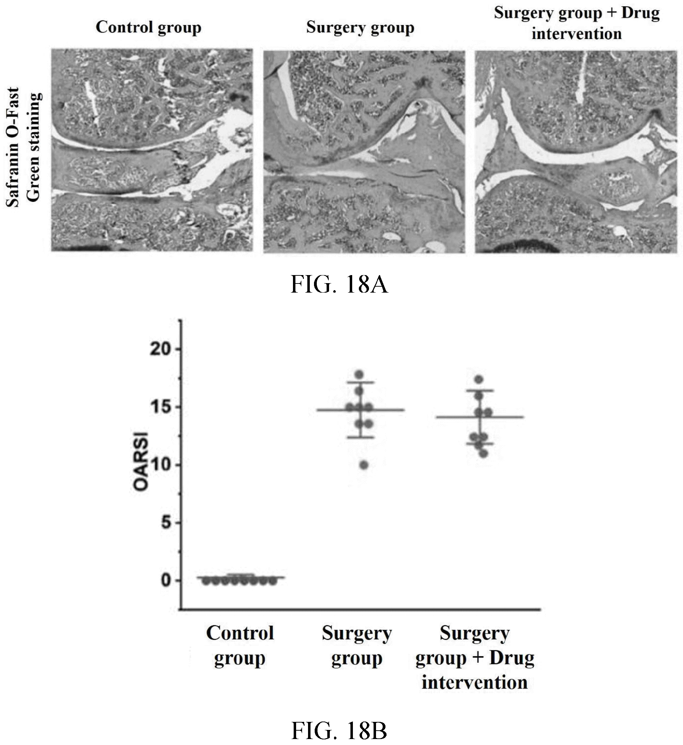

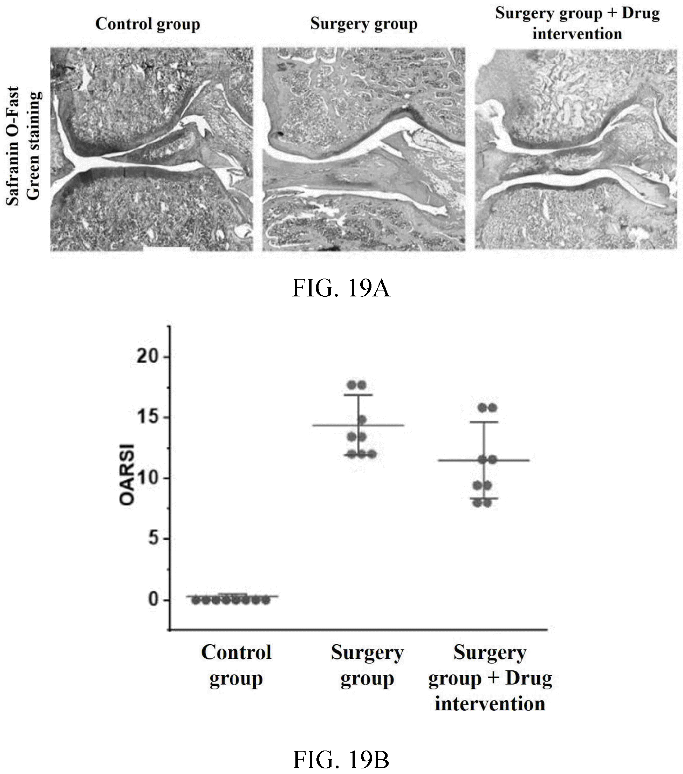

Representative results of the control molecule and the PACE4 inhibitory peptides in the examples of the present disclosure were randomly and exemplarily shown in FIGs. 18A-B and FIGs. 19A-B.

It could be observed that in the test for preventive efficacy, Safranin O-Fast Green staining results showed that the PACE4 inhibitory peptides in the examples of the present disclosure could effectively prevent surgery-induced cartilage degeneration and severe GAG (i.e., containing negatively charged carboxyl and sulfate groups, mainly released by osteoblast-like cells (BNCs) stimulated by IL-1) loss.

### Example 8: Therapeutic effect of long-term administration of PACE4 inhibitory peptide

In addition, the effect of different administration frequencies on therapeutic efficacy was further tested. A total of 24 SD rats were divided into three groups, including a control group, an OA modeling group (surgery group), and an OA modeling + therapeutic administration group (surgery group + drug intervention), with 8 rats in each group. In this example, Ac-dR-R-A(4-Pip)-K-R-dT-NH₂ was used as a representative peptide to exemplarily demonstrate the similar effects of each PACE4 inhibitory peptide in the examples of the present disclosure. The administration frequency of Ac-dR-R-A(4-Pip)-K-R-dT-NH₂ was changed from 2 times (intra-articular injection on day 3 and day 10 after MMT surgery) to 4 times (intra-articular injection on days 3, 7, 10 and 24 after MMT surgery). Rats in each group were sacrificed on day 31 after modeling, and joint tissues were collected. Safranin O-Fast Green staining, OARSI scoring, and TRAP staining were performed according to the methods described in the above examples to determine the subchondral bone resorption in rats before and after MMT surgery and during treatment with the PACE4 inhibitory peptide.

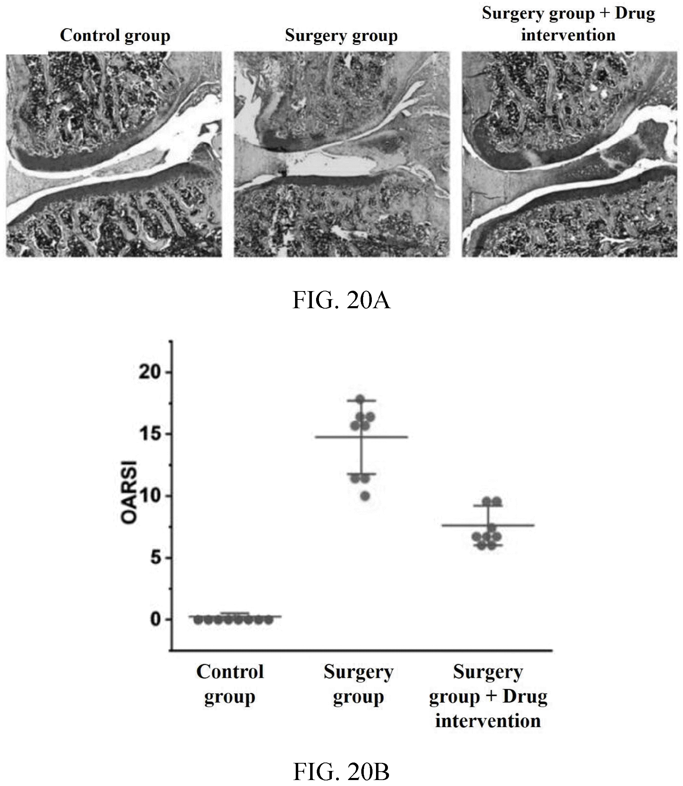

The results of the representative PACE4 inhibitory peptide Ac-dR-R-A(4-Pip)-K-R-dT-NH₂ were exemplarily shown (FIGs. 20A-B).

From the above tests, it could be observed that compared to the control group, the OARSI score was significantly upregulated after OA modeling, but was downregulated in the PACE4 inhibitory peptide treatment group. Compared to the OA group, in the preventive test, the OARSI score was reduced by 20% in the intervention group treated with peptide Ac-dR-R-A(4-Pip)-K-R-dT-NH₂, whereas the OARSI score was reduced by only 4% in the intervention group treated with the control peptide Ac-R-F-K-R-NH₂, showing no obvious preventive effect. In the therapeutic test, the OARSI scores were reduced by 28%, 34.6%, 40.2%, and 38.5% in the intervention groups treated with Ac-dR-R-F-K-R-dT-NH₂, Ac-dR-R-F(4-CF₃)-K-R-dT-NH₂, Ac-dR-R-A(4-Pip)-K-R-dT-NH₂, and Ac-dR-R-F(4-Guan)-K-R-dT-NH₂, respectively, whereas the OARSI score was reduced by only 10% in the intervention group treated with the control peptide Ac-R-F-K-R-NH₂, showing no obvious therapeutic effect. Meanwhile, in the 4-administration treatment test over 24 days, the OARSI score was reduced by 48.1% in the intervention group. The above results indicated that the PACE4 inhibitory peptides had significant preventive and therapeutic effects (the therapeutic effect was relatively more significant).

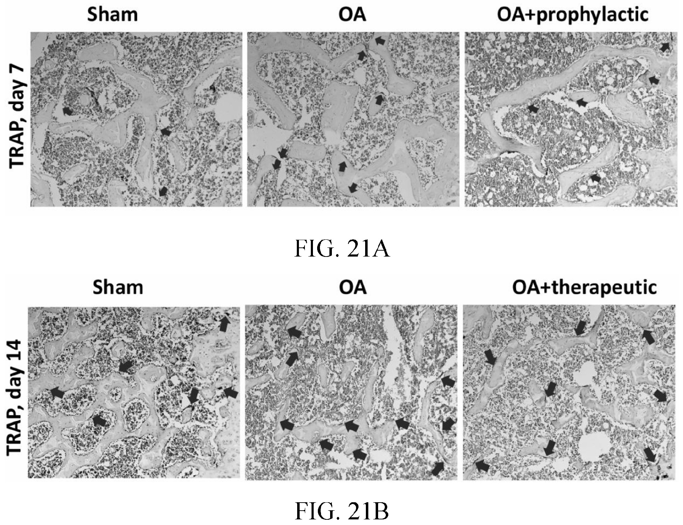

Tartrate-resistant acid phosphatase (TRAP) is a specific marker enzyme for osteoclasts. TRAP staining of bone tissue can reveal the extent of bone resorption of osteoclasts. TRAP staining results (FIGs. 21A-B) showed no significant differences between groups in the arthritis prevention test, whereas in the therapeutic test, TRAP-positive deposits were slightly increased in the OA group and improved under Ac-dR-R-A(4-Pip)-K-R-dT-NH₂ treatment. Therefore, it could be concluded that administration of Ac-dR-R-A(4-Pip)-K-R-dT-NH₂ on day 14 after MMT surgery slightly inhibited osteoclast formation in subchondral bone.

In summary, the PACE4 inhibitory peptides in the examples of the present disclosure exhibited relatively strong therapeutic and preventive effects on cartilage degeneration.

The above embodiments are preferred embodiments of the present disclosure, but the embodiments of the present disclosure are not limited to the above embodiments. Any changes, modifications, substitutions, combinations, or simplifications made without departing from the spirit and principle of the present disclosure shall be considered equivalent substitutions and shall be included within the protection scope of the present disclosure.

## Claims

1. A polypeptide or a derivative thereof, wherein the polypeptide has a general formula shown in formula (I):
Arg-AA2-AA3-Arg-AA4 formula (I),
wherein,
AA2 is selected from the group consisting of Phe and Ala;
AA3 is selected from the group consisting of Arg and Lys; and
AA4 is Thr, or AA4 is absent.

2. The polypeptide or the derivative thereof according to claim 1, wherein the polypeptide is:
AA1-Arg-AA2-AA3-Arg-AA4;
wherein AA1 is selected from the group consisting of Arg, Pro, and a combination thereof.

3. The polypeptide or the derivative thereof according to claim 1 or 2, wherein an amino acid in the polypeptide or the derivative thereof is in D configuration or L configuration.

4. The polypeptide or the derivative thereof according to claim 1 or 2, wherein at least 3 basic amino acids are present in the polypeptide or the derivative thereof.

5. The polypeptide or the derivative thereof according to claim 1 or 2, wherein a modification of an intermediate residue is present in the polypeptide or the derivative thereof.

6. The polypeptide or the derivative thereof according to claim 5, wherein the modification of an intermediate residue is a chemical modification, preferably comprising 4-trifluoromethyl modification, 4-guanidino modification and 4-methylpiperidine modification.

7. The polypeptide or the derivative thereof according to claim 5 or 6, wherein the intermediate residue is AA2.

8. The polypeptide or the derivative thereof according to any one of claims 1 to 7, wherein the derivative comprises a pharmaceutically acceptable salt and an isomer.

9. The polypeptide or the derivative thereof according to claim 8, wherein the pharmaceutically acceptable salt comprises at least one of a metal salt, an ammonium salt, a salt formed with an organic base, a salt formed with an inorganic acid, and a salt formed with an organic acid.

10. The polypeptide or the derivative thereof according to claim 8, wherein the isomer comprises a stereoisomer and a tautomer.

11. A peptide conjugate, comprising the polypeptide or the derivative thereof according to any one of claims 1 to 10 and a modification moiety, wherein the modification moiety is located at the N-terminus and/or C-terminus of the polypeptide or the derivative thereof.

12. The peptide conjugate according to claim 11, wherein the modification moiety comprises at least one of a chemical modification, a targeting moiety, a fluorescent dye and a protein tag.

13. The peptide conjugate according to claim 12, wherein the chemical modification comprises at least one of amidation, acetylation, amination, methylation, phosphorylation, glycosylation, lipidation, and ubiquitination.

14. The peptide conjugate according to claim 11, wherein the peptide conjugate comprises:
Ac-R-F-K-R-NH₂
Ac-R-F-K-R-dT-NH₂
Ac-R-F-K-R-T-NH₂
Ac-R-R-F-K-R-NH₂
Ac-dR-R-F-K-R-dT-NH₂
P-dR-R-F-K-R-dT-NH₂
dP-dR-R-F-K-R-dT-NH₂
Ac-R-F(4-CF₃)-K-R-T-NH₂
Ac-R-F(4-CF₃)-K-R-dT-NH₂
Ac-dR-R-F(4-CF₃)-K-R-dT-NH₂
Ac-dR-R-F(4-CF₃)-K-R-T-NH₂
P-dR-R-F(4-CF₃)-K-R-dT-NH₂
dP-dR-R-F(4-CF₃)-K-R-dT-NH₂
Ac-R-A(4-Pip)-K-R-dT-NH₂
Ac-dR-R-A(4-Pip)-K-R-dT-NH₂
Ac-R-R-A(4-Pip)-K-R-dT-NH₂
Ac-dR-R-A(4-Pip)-K-R-dT-NH₂
P-dR-R-A(4-Pip)-K-R-dT-NH₂
dP-dR-R-A(4-Pip)-K-R-dT-NH₂
Ac-R-F(4-Guan)-K-R-dT-NH₂
Ac-dR-R-F(4-Guan)-K-R-dT-NH₂
Ac-R-R-F(4-Guan)-K-R-dT-NH₂
Ac-dR-R-F(4-Guan)-K-R-T-NH₂
P-dR-R-F(4-Guan)-K-R-dT-NH₂
dP-dR-R-F(4-Guan)-K-R-dT-NH₂.

15. A nucleic acid molecule, comprising:
(1) a nucleic acid molecule encoding the polypeptide or the derivative thereof according to any one of claims 1 to 10, or the peptide conjugate according to any one of claims 11 to 14; or
(2) a sequence having at least 70% sequence identity with (1), and the sequence retains PACE4 inhibitory activity.

16. The nucleic acid molecule according to claim 15, wherein the nucleic acid molecule contains a modified base.

17. A biomaterial comprising or expressing any one of the polypeptide or the derivative thereof according to any one of claims 1 to 10, the peptide conjugate according to any one of claims 11 to 14, and the nucleic acid molecule according to claim 15 or 16, wherein the biomaterial comprises an expression cassette, a vector, and a cell.

18. A method for preparing the polypeptide or the derivative thereof according to any one of claims 1 to 10, or the peptide conjugate according to any one of claims 11 to 14, comprising obtaining the same by using the biomaterial according to claim 17, or by solid-phase synthesis.

19. A PACE4 inhibitor, comprising the peptide conjugate according to any one of claims 11 to 14, and a pharmaceutically acceptable excipient and/or carrier.

20. A pharmaceutical composition, comprising the peptide conjugate according to any one of claims 11 to 14 and at least one second pharmaceutically active ingredient.

21. The pharmaceutical composition according to claim 20, wherein the second pharmaceutically active ingredient comprises an anti-inflammatory drug, a cartilage-nourishing agent, an analgesic, and a hormone.

22. Use of the polypeptide or the derivative thereof according to any one of claims 1 to 10 and/or the peptide conjugate according to any one of claims 11 to 14 in the preparation of a drug for anti-osteoarthritis.

23. The use according to claim 22, wherein a dosage form of the drug comprises at least one of powder, tablet, granule, capsule, sustained-release formulation, solution, dry suspension, emulsion, suspension, syrup, and drop.

24. The use according to claim 22, wherein an applicable subject of the drug comprises a subject and a patient.

25. The use according to claim 24, wherein the subject comprises a non-human animal and a human.

26. The use according to claim 22, wherein the anti-osteoarthritis comprises treatment and/or prevention of osteoarthritis.
